# EUROPEAN PATENT APPLICATION

(11) **EP 1 192 896 A1**
(43) Date of publication of application: **03.04.2002**
(21) Application number: 00931660.5
(22) Date of filing: 02.06.2000
(51) Int. Cl.: A61B 5/00, A61B 10/00

(54) **RISK REDUCTION TABLE, METHOD FOR CREATING THE SAME, RISK CARE SET INCLUDING RISK REDUCTION TABLE, AND RISK CARE BUSINESS SYSTEM**

(30) Priority: 04.06.1999 JP 15854999
(71) Applicant: Sunstar Inc., Takatsuki-shi, Osaka 569-1134 (JP)
(72) Inventor: TAKEMURA, Akane, Ibaraki-shi, Osaka 567-0831 (JP); YASUDA, Naomi, Ibaraki-shi, Osaka 567-0021 (JP); TAKASE, Naoko, Takatsuki-shi, Osaka 569-1141 (JP)
(74) Representative: Lemoine, Robert
(86) International application number: JP0003572
(87) International publication number: WO0074559

(57) **Abstract**

An object of the present invention is to provide a risk improvement table allowing calculation of objective risk values not depending on the experience or knowledge of the person making the judgement, and examples of applications using the risk improvement table. A risk improvement table has been constructed that has a 'risk estimation' processing part allowing risk values to be calculated through answering questions, and a 'risk avoidance measure presentation' processing part that presents specific measures for avoiding or improving the judged risk in accordance with the contents of this risk. Moreover, a risk care business system in which the risk improvement table is combined with examination means and risk improvement tools has also been constructed.

## Description

### BACKGROUND OF THE INVENTION

### 1. Field of the Invention

The present invention relates to a risk improvement table comprising a 'risk estimation' processing part and a 'risk avoidance measure presentation' processing part, which predicts possibility of oral diseases such as caries and periodontal disease in an objective and overall fashion, and presents optimum measures for avoiding the predicted overall risk of caries, periodontal disease and the like, without the extent of progression of the diseases being judged based on the knowledge and experience of a specialist such as a dentist as done conventionally. The present invention also proposes providing the risk improvement table with display parts for the predicted overall risk and the optimum avoidance measures, and an improvement possibility display part that displays the possibility of improvement predicted from the avoidance measures, meaning that the risk improvement table can be used as a tool for assisting in evoking and encouraging preventative action. Moreover, the present invention also proposes a risk care set, in which are combined the above risk improvement table and risk improvement tools, and which thus has risk avoidance or improvement effects, and a risk care method using this risk care set. Furthermore, the present invention also discloses a technical idea common to the risk improvement table, the risk care set and the risk improvement method selected when inventing the risk improvement table, the risk care set and the risk improvement method, namely a risk judgement method.

### 2. Description of the Related Art

It goes without saying that early discovery and early treatment are desirable with all diseases, and it is also important to spot possibility of a disease before the disease is contracted. This can be said for all medical fields including the field of oral medicine, and in the field of oral medicine prediction of the outbreak and progression of diseases has been carried out hitherto by specialists such as dentists, although this has tended to rely greatly on the experience and knowledge of the dentists.

On the other hand, there is also research into predicting the outbreak and progression of diseases objectively. In this research, the risk possibility of contracting a disease or the risk possibility of a disease progressing in extent is ascertained through the concept of 'risk', and factors relating to the risk and factors causing the disease (hereinafter referred to as 'risk factors') are analyzed, thus trying to get a grasp upon possibility of outbreak or progression of the disease. In the field of oral medicine, data is obtained from examination of the oral cavity to gain information relating to risk factors such as the amount of plaque on the teeth, the color of the gums and the straightness of the teeth, questioning of the patient about risk factors such as the number of times that he/she brushes his/her teeth per day and whether or not he/she smokes, and the like, and from this data an attempt is made to predict the risk possibility of oral diseases being contracted or progressing, i.e. the overall 'risk'.

For example, there is a method of predicting the risk of caries in which data is gathered on a test subject, and calculations are carried out following clearly stated judgement criteria, thus judging the risk towards the disease in question (Department of Community Dentistry, University of Texas Health Science Center at San Antonio. M.W.J. Dodds et al., J. Dent. Educ. 1995, 59, 945-956). This research has been carried out primarily for academic purposes, and although some attempts to utilize the results have been made in health examinations and at hospitals, the contents of the examination of the oral cavity and the questioning of the test subject carried out to gather the data are complex, and hence the method is not fit for practical use.

Moreover, there are also file-format notes for judging risk called 'My Teeth's Health Notes' (Morimura Corporation) in which gathered data relating to risk factors is shown visually using a radar chart and information relating to caries and periodontal disease is shown, but objective criteria for judging the overall risk are not clearly stated, and hence when judging the overall risk the experience and knowledge of the person making the judgement inevitably has a large influence, and the notes can only be used by a specialist such as a dentist.

Moreover, depending on the disease, the outbreak and progression of a disease may be closely related to lifestyle habits, and in the field of oral medicine, for which this relationship is particularly close, guidance on improving lifestyle habits is adopted as a measure for suppressing the outbreak and progression of disease, but there are situations in which such guidance tends not to be effective. In other words, even in the case of the two big oral diseases, caries and periodontal disease, it is known that improving lifestyle habits such as eating habits and oral hygiene habits is one of the main preventative methods, but it is hard to communicate the importance of this, and there thus tends to be a lack of awareness regarding improving lifestyle habits or continuing positive preventative measures, and as a result guidance on making such improvements tends not to be effective.

In this way, with regard to prevention of oral diseases, practical criteria enabling people not having specialist knowledge to get a grasp upon 'risk' and judge the overall risk objectively, and enabling presentation of optimum risk avoidance measures (measures for reducing the probability of contracting a disease, or in the case that a disease has already been contracted slowing down the progression of the condition or improving the condition) in accordance with the risk situation, have not yet been established, and hence there is a problem that it is difficult to make people take risk avoidance measures continually.

In view of the present state of affairs as described above, the present invention aims to provide a practical risk improvement table, which allows overall risk to be judged objectively without depending on the experience and knowledge of the person judging the risk, which is capable of presenting suitable risk avoidance measures or lifestyle habit improvement measures based on the judgement results obtained, and which is capable of carrying out a display for evoking and encouraging continuous implementation of these measures. Moreover, the present invention also discloses a method of creating this beneficial risk improvement table, and also proposes a risk care set including the risk improvement table, and a system for carrying out risk care business.

The present invention does not stop merely at preventing oral diseases, but rather aims at further promoting health, and, with rapidly rising medical costs having become a social problem, can be said to be something that has been long awaited by society.

### SUMMARY OF THE INVENTION

The present inventors carried out assiduous studies, and eventually as a result accomplished a risk improvement table comprising a 'risk estimation' processing part and a 'risk avoidance measure presentation' processing part, and moreover by providing display parts that display the overall risk and risk avoidance measures and the possibility thereof, accomplished a risk improvement table that can be utilized as a means for evoking and encouraging preventative measures.

Specifically, a first object of the present invention is to provide a risk improvement table, enabling objective risk judgement not affected by specialist knowledge or experience, and enabling suitable risk improvement guidance to be carried out based on the judgement results obtained. The risk improvement table is provided with the following two processing parts (1) and (2).
(1) 'Risk estimation' processing part: A part for carrying out processing for judging risk factors, risk values and an overall risk value accurately and objectively. Question items and examination items for identifying the risk factors, for example, are listed in this processing part, and the processing part is devised such that an overall risk value can be obtained by arranging the results of the questioning and the examination in accordance with a processing procedure indicated in this processing part.
(2) 'Risk avoidance measure presentation' processing part: A part for carrying out processing relating to identifying measures for reducing the 'risk' which is judged unequivocally from the overall risk value. In this part, for example risk factors are displayed, and specific measures that can be taken for reducing the risk due to these risk factors are shown.

A second object of the present invention is to provide a risk improvement table provided with display parts that display the overall risk and risk avoidance measures and the possibility thereof, thus evoking and encouraging preventative action.

A third object of the present invention is to apply a risk improvement table as described above to the field of oral medicine in particular.

A fourth object of the present invention is to provide a risk care set comprising the above risk improvement table and tools for improving risk (hereinafter referred to as 'risk improvement tools'). That is, using optimum risk improvement tools based on an objective risk judgement is a first step in realizing excellent improvement effects, and hence the risk care set combines the risk improvement table and risk improvement tools.

A fifth object of the present invention is to provide a method of improving risk using either the above risk improvement table or the above risk care set.

A sixth object of the present invention is to indicate a procedure for creating the above risk improvement table, i.e. a creation method. The disclosure of this creation procedure is necessary for understanding the significance of each part of the risk improvement table. Moreover, this procedure is the same for all diseases, and hence by understanding this procedure a risk improvement table can be created for any of various specific diseases.

A seventh object of the present invention is to propose an overall system for when developing risk care business using the above risk improvement table.

The risk improvement table of the present invention is based on the above ideas. Of particular importance is that the overall risk judgement can be carried out objectively, and optimum risk avoidance measures are presented in accordance with this. The risk improvement table is thus provided with processing parts having the following functions (1), (2) and (3).
(1) From the standpoint of practicality and convenience, clear specification of objective criteria for identifying risk factors and risk values thereof.
(2) Determination of an overall risk value capable of being determined unequivocally from the result of arithmetic processing on the risk values for the risk factors.
(3) Judgement criteria for unequivocally displaying the relationship between the overall risk value and possibility of the targeted disease being contracted or progressing.

In the method of creating the risk improvement table of the present invention, when judging the risk based on the created risk improvement table, it is important that necessary examinations are as simple as possible without the reliability thereof being reduced. To make this possible, firstly it is necessary to reduce the number of risk factors targeted for investigation, and to make the examination items for identifying whether or not these risk factors are possessed or the risk values thereof simple so as to be suitable for implementation in a clinical setting. The risk improvement table is created based on these ideas through the following operations.
(1) Identify the principal risk factors for the targeted oral disease.
(2) Eliminate ones of the identified risk factors that are known to be correlated to other ones of the risk factors.
(3) Calculate the risk value, which represents the rate of contribution to disease outbreak, for each of the risk factors remaining after the above operation.
(4) Establish the relationship between an overall risk value calculated from the risk values for the risk factors and the state of progression of the oral disease.
(5) Identify question-based examination items and oral cavity examination items necessary for judging whether or not a test subject has these risk factors, and of those identified question-based examination items and oral cavity examination items, as far as possible replace items that are unsuitable for implementation in a clinical setting with other items correlated thereto, thus adjusting the items so that only items suitable for implementation in a clinical setting remain.

A risk improvement table created in this way can be used for everyone.

Moreover, when developing risk care business using the risk improvement table, a risk care business system is constructed in which the risk improvement table is combined with examination means for implementing the examination items written in the risk improvement table and risk improvement tools for implementing the risk avoidance measures presented by the risk improvement table.

### BRIEF DESCRIPTION OF THE DRAWINGS

FIG. 1 is an explanatory view showing a first sheet of a risk improvement table;
FIG. 2 is an explanatory view showing a second sheet of the risk improvement table;
FIG. 3 is an explanatory view showing a third sheet of the risk improvement table;
FIG. 4 is an explanatory view showing a fourth sheet of the risk improvement table; and
FIG. 5 is a relationship diagram showing the relationship between the various parts and sections of the risk improvement table.

### DESCRIPTION OF THE PREFERRED EMBODIMENTS

Before describing embodiments of the present invention, an explanation will be given of the relationship between risk, risk factors, risk values, and the overall risk value.

As already stated, 'risk' refers to the risk possibility of a disease being contracted or progressing in extent. 'Risk factors ' refers to factors relating to the risk of oral diseases. 'Risk values' are numerical values assigned unequivocally for each risk factor based on answer values. Arithmetic processing is carried out on these risk values to obtain an overall risk value.

A risk improvement table according to the present invention is created either for each oral disease individually or for a combination of two or more oral diseases. These diseases include, but are not limited to, caries such as root caries, secondary caries, incipient caries and deciduous caries, periodontitis (alveolar pyorrhea), gingivitis, hypersensitivity, temporomandibular arthrosis, oral cancer, stomatitis, mastication abnormalities, malocclusion, xerostomia, salivary gland abnormalities, and halitosis.

A description will now be given of the 'risk estimation' processing part. There are no particular limitations on the format of the 'risk estimation' processing part, provided it is possible to collect data on the current state with regard to the risk factors in a format enabling conversion to risk values (numerical data), and is possible to estimate the overall risk from this current state data.

The risk values are analytic values based on past statistical data, and are allocated for each of a plurality of fixed levels for each of the selected risk factors. That is, each risk value reflects the extent to which the current state with regard to the risk factor in question contributes to the risk of disease.

There are no particular limitations on the format of the 'risk estimation' processing part, but a format can be adopted, for example, in which the 'risk estimation' processing part has: a data gathering part provided with an examination item display section that displays 1 to 30, preferably 5 to 15, risk factors and means for examining the presence/absence or current state of these risk factors, and an answers section corresponding to the examination item display section; a judgement criteria part provided with a risk value judgement criteria section that stipulates an unequivocal correlation between the answers and preset risk values; a risk judgement part provided with a judgement section for recording the risk values for the risk factors converted from the answers based on the risk value judgement criteria section and calculating an overall risk value by arithmetic processing; and an overall risk display part provided with an overall risk judgement criteria section that unequivocally stipulates an overall risk allocated to one of a plurality of preset levels from the overall risk value.

The 'risk avoidance measure presentation' processing part is a part for presenting suitable risk avoidance measures pre-allocated for each of the levels of the overall risk and/or for risk factors that contribute greatly to the risk.

The overall risk estimated through the 'risk estimation' processing part is displayed in the overall risk display part. The various levels of the overall risk may be represented by numbers, for example 'level 1', 'level 2' etc., but it is preferable to use perceptualization means using diagrams from the standpoint of making intuitive understanding possible. Moreover, an explanation section in which the extent of the risk is explained may be provided.

The risk avoidance measures are displayed in a risk avoidance measure display part. Here, it is preferable to provide not only a means display section for explaining improvement means, but also a guidance display section for explaining improvement guidance measures. Here, 'improvement means' refers to means that are used or implemented for improving the risk, for example medicaments or implements effective in improving the risk. Moreover, 'improvement guidance measures' refers to advice or guidance on lifestyle habits, attitudes and the like.

Furthermore, to evoke and encourage continuous implementation of the improvement means, it is preferable to provide an improvement possibility display part.

Risk factors will now be described in detail. There are no limitations on the risk factors. Risk factors are selected for each disease based on the relationship between the risk factor and the disease, and vary according to the type of disease and the age (generation) of the test subject. Moreover, if once risk factors are selected, these risk factors are used continuously. However the risk factors are reconsidered about every 4 or 5 years. Such reconsideration of the risk factors is carried out so that the appearance of new scientific theories, the discovery of new risk factors, new proposals on methods of judging risk factors and so on are reflected in the risk improvement table.

The risk factors are selected from risk factors relating to all kinds of oral diseases, and can be broadly classified into a group relating to oral diseases in general and a group relating to specific oral diseases. In the present invention, about 1 to 30 factors, preferably 5 to 15 factors, are selected from the oral diseases in general group and/or the specific oral diseases group and used as the risk factors.

Risk factors relating to oral diseases in general include:
1) Individual factors: Sex, age, medical history, nutritional status, etc.
2) State of oral hygiene: Amount of plaque deposited on teeth, areas where plaque is deposited, amount of plaque deposited in specific areas (in the case of a child, the state of oral hygiene of the child-rearer is also included), etc.
3) Oral hygiene habits: frequency of brushing teeth, method of brushing teeth, whether or not implements for cleaning between teeth (interdental brushes, dental floss etc.) are used, whether or not periodic check-ups are received, whether or not periodic visits to a dental clinic are made, whether or not tooth surface cleaning is carried out by a specialist, etc.
4) Awareness of oral hygiene: Awareness of oral hygiene habits, social/environmental factors influencing oral hygiene habits, etc. (in the case of a child, the awareness of oral hygiene of the child-rearer is also included).
5) General health care awareness: Factors relating to health care awareness other than oral, factors relating to lifestyle habits, social/environmental factors influencing lifestyle habits, etc. (in the case of a child, the general health care awareness of the child-rearer is also included).
6) Morphology of teeth: State of dentition, state of prostheses, state of conformity at margin with prostheses, whether or not denture used, presence/absence of orthodontic devices, etc.
7) Saliva: Amount of saliva secretion (mouth dryness), glucose clearance, saliva buffer capacity, amount of components in saliva acting upon bacteria, amount of components in saliva acting upon teeth, etc.

Moreover, risk factors relating to a specific oral disease include: In the case of caries:
1) Cariogenic bacteria: Numbers of bacteria, composition of bacteria, plaque acid production capacity, etc. (in the case of a child, cariogenic bacteria of the child-rearer is also included).
2) Sugar intake (eating habits): Amount and frequency of sugar intake, food and drink preferences, amount and frequency of intake of foods and drinks having a high sugar content, amount and frequency of food and drink intake, amount and frequency of food and drink intake other than meals, amount and frequency of food and drink intake other than regular between-meal snacks and meals, amount and frequency of food and drink intake before bed, presence/absence of intake of special foods; moreover, in the case of breast-feeding children, whether or not feeding bottle used, contents of feeding bottle, whether or not breast-feeding carried out or feeding bottle used before bed, contents of feeding bottle used before bed (in the case of a child, sugar intake (eating habits) of the child-rearer is also included).
3) Properties of teeth: Acid resistance of teeth, hardness of teeth, amount of retained fluoride in teeth, crystal structure of teeth, surface properties of teeth, time passed since eruption of teeth, presence/absence of deciduous teeth, presence/absence of subsurface lesions, whether or not root surface exposed, whether or not fluoride used, whether or not sealant treatment carried out.
4) Morphology of pits and fissures: Depth of pits and fissures, complexity of pits and fissures.
5) Caries experience: Number of teeth having caries experience, number of tooth surfaces having caries experience, whether or not caries experience of specific tooth surfaces, caries incidence pattern (in the case of a child, caries experience of the child-rearer is also included).

In the case of periodontal disease:
1) Periodontopathic bacteria: Numbers of periodontopathic bacteria, substances produced by periodontopathic bacteria.
2) Smoking: Whether or not currently smoke, whether or not experience of smoking in past, number of cigarettes smoked, number of years been smoking, number of pack-years (no. cigarettes per day x no. years been smoking).
3) Alcohol intake: Presence/absence of alcohol abuse, amount of alcohol intake, frequency of alcohol intake.
4) Stress: Presence/absence of financial problems, whether or not socially isolated, whether or not depressed, presence/absence of socio-psychological factors related to anger, presence/absence of chronic stress, ability to cope with stress.
5) Genetics: Genetic type of IL-1-related genes, genetic type of TNF-related genes, symptoms related to age, sex.
6) Specific systemic diseases: Osteoporosis and related symptoms, diabetes and related symptoms, HIV and related symptoms, hypertension and related symptoms, medication for the above.
7) Past history of periodontal disease: Whether or not experienced treatment for periodontal disease, whether or not experienced periodontal surgery, extent of reaction to treatment for periodontal disease.

A description will now be given of risk improvement tools used in the present invention. Risk improvement tools comprise guidance tools and/or products for the oral cavity such as medicaments for the oral cavity and implements for the oral cavity. There are no particular limitations on the form of a guidance tool, provided the guidance tool is capable of achieving in general aims such as teaching of knowledge and increasing of awareness with regard to oral hygiene and diseases. Specifically, guidance tools include videos, CDs, cassette tapes, panels, books, computer software and the like containing guidance on improving eating habits, brushing guidance and the like, or alternatively these guidance tools can be integrated with a device such as a display.

Products for the oral cavity include commercially available mouthwashes, sprays, foams, gels, tablets, chews, capsules, gum, foods, toothbrushes, interdental brushes, dental floss, and irrigators. Specific examples include foams for strengthening dentine, varnishes for preventing bacterial infection, foods for strengthening teeth and bones, chews for preventing mouth dryness, capsules for reducing damage due to smoking, tablets that supplement components in the saliva, dental floss for killing bacteria in specific areas, interdental brushes for removing plaque from specific areas, irrigators for washing specific areas, and capsules for improving the patient's resistance.

The risk improvement tools used will change according to the risk factors. It is preferable for both the risk factors possessed by the test subject and risk improvement tools used for improving these risk factors to be indicated in the risk improvement table. The indication of the risk improvement tools in the risk improvement table may be carried out using either generic terms such as 'fluoride toothpaste' or specific product names such as '... made by ...'.

The risk improvement table of the present invention may be provided using a medium such as paper, in which case the risk improvement table is filled in using a writing implement. Alternatively, the whole or part of the display or processing contents of each of the sections that constitute the risk improvement table may be realized using a computer program. In this case, display on paper becomes display on a display screen, and filling in using a writing implement becomes filling in using a keyboard or a pointing device such as a mouse. The computer input may be carried out from a remote location via a communications network such as the internet. In this case, the risk improvement table may be made to be downloadable from a website, or may be made to be such that the risk improvement table is displayed online using a web browser and filled in.

The present invention will now be described in yet more detail through an embodiment.

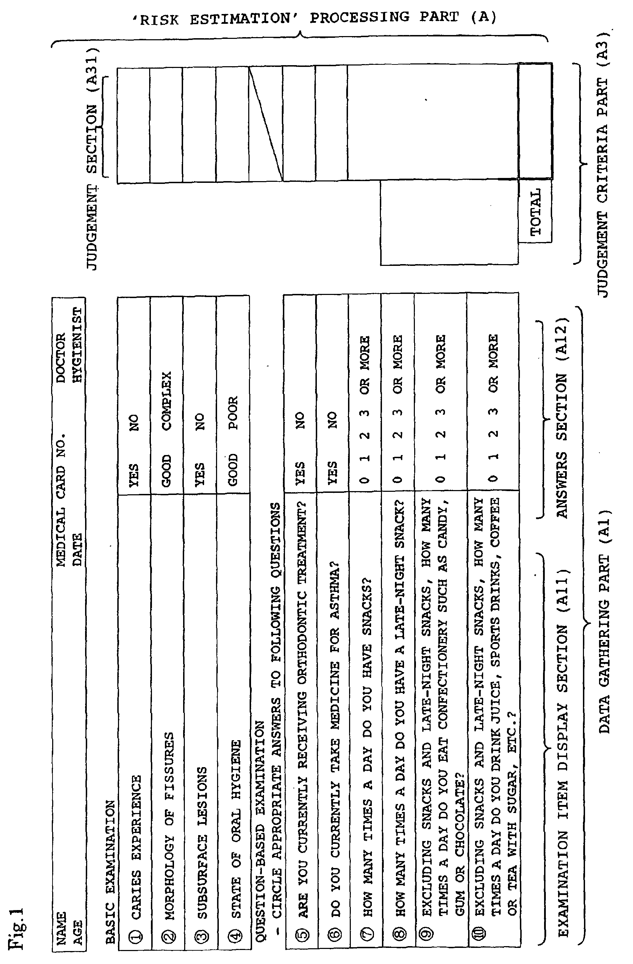

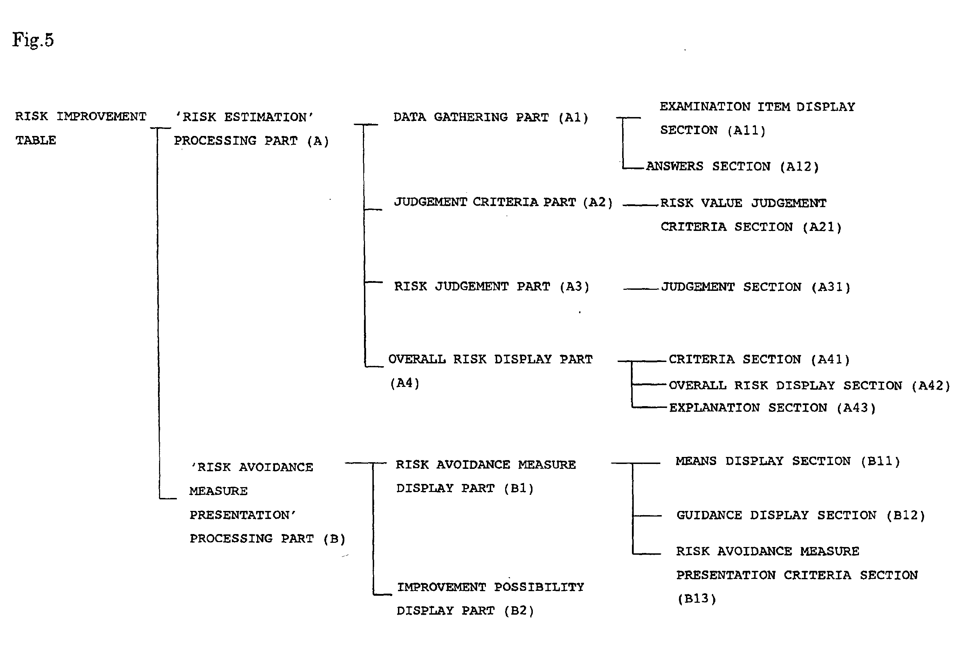

FIGS. 1 to 4 show an embodiment of the risk improvement table of the present invention, and FIG. 5 is a relationship diagram showing the relationship between the various parts and sections shown in FIGS. 1 to 4. The risk improvement table of the present embodiment targets 'caries', but the targeted disease is not limited to this and may be something else.

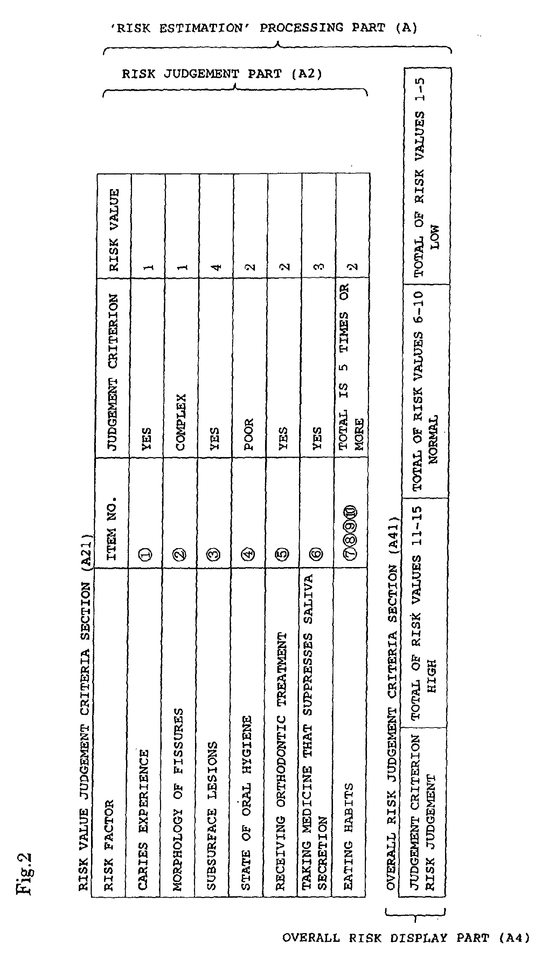

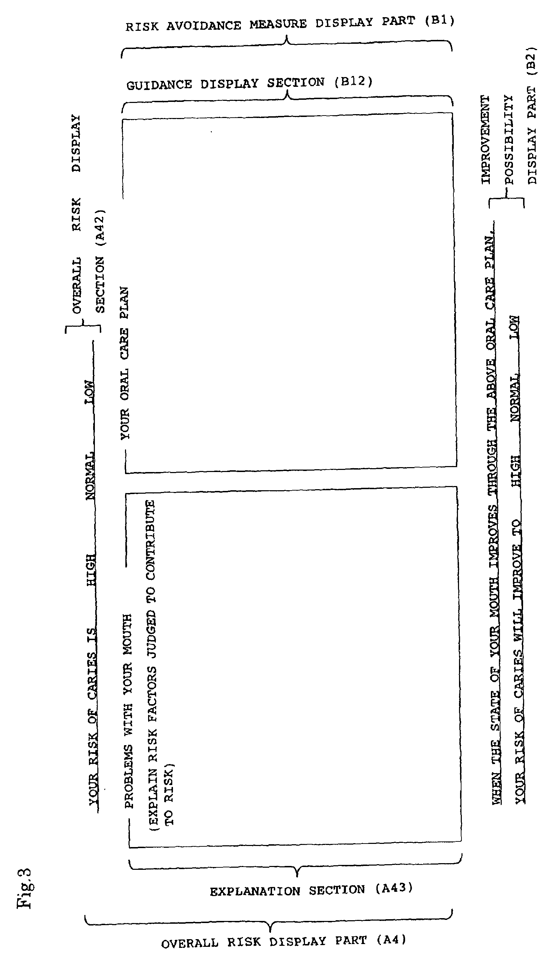

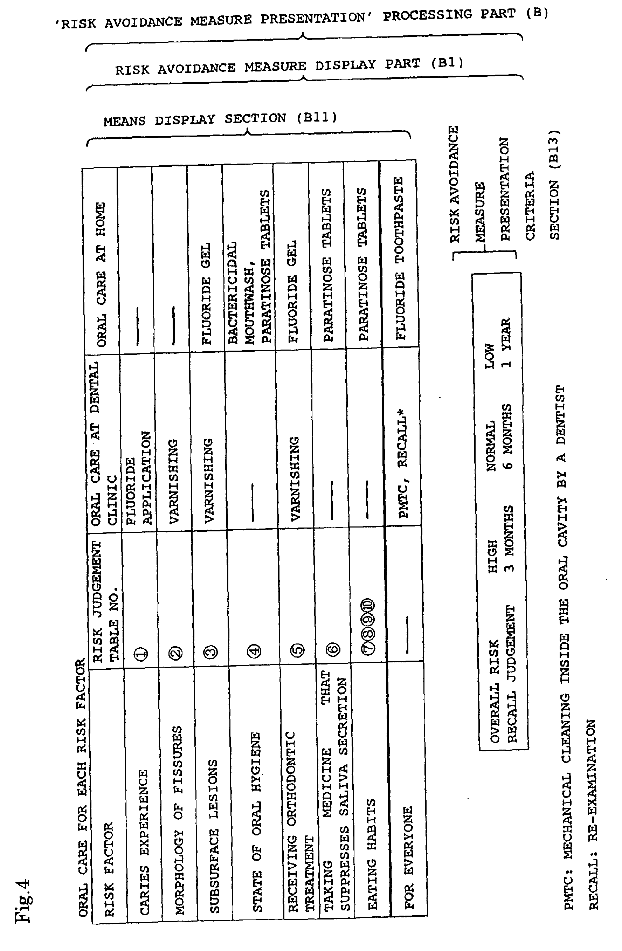

Following is a description of the risk improvement table shown in FIGS. 1 to 4, with reference to the relationship diagram shown in FIG. 5. Here, the sheet shown in FIG. 1 is handled as sheet 1, the sheet shown in FIG. 2 as sheet 2, the sheet shown in FIG. 3 as sheet 3, and the sheet shown in FIG. 4 as sheet 4. However, the sheets may be combined into one.

As shown in FIG. 5, broadly divided, the risk improvement table is composed of a 'risk estimation' processing part A and a 'risk avoidance measure presentation' processing part B. The 'risk estimation' processing part A is a part for carrying out processing for identifying risk factors and risk values, and displaying precise, objective criteria for judging the overall risk which is obtained from the identified risk values, and means for doing the same. The 'risk avoidance measure presentation' processing part B, on the other hand, is a part for presenting optimum avoidance means in accordance with the 'risk' determined unequivocally from the overall risk value, i.e. the risk possibility of the targeted disease being contracted or progressing in extent.

In the present example, all of FIGS. 1 and 2 and part of FIG. 3 correspond to the 'risk estimation' processing part A, and the remainder of FIG. 3 and all of FIG. 4 correspond to the 'risk avoidance measure presentation' processing part B. Following is a description of the contents of the 'risk estimation' processing part A and the 'risk avoidance measure presentation' processing part B.

The 'risk estimation' processing part A is provided with a data gathering part A1 for gathering data to be used in the risk judgement, a judgement criteria part A2 for judging the risk based on the gathered data, a risk judgement part A3 that provides criteria for the judgement, and an overall risk display part A4 for displaying the overall risk calculated from the risk values.

The data gathering part A1 has a question format, being composed of an examination item display section A11 and an answers section A12, and being devised such that the data necessary for the risk judgement can be gathered by answering questions. Although, basically speaking, the data is gathered by answering questions in this way, for items for which it is judged that it is appropriate to carry out a detailed examination, an examination such as an examination of the oral cavity may be carried out instead of the questioning. The examination items are thus divided into a basic examination and a question-based examination.

The judgement criteria part A2 is provided with a risk value judgement criteria section A21, the risk judgement part A3 is provided with a judgement section A31, and the overall risk display part A4 is provided with an overall risk judgement criteria section A41, an overall risk display section A42 and an explanation section A43. It is important in the present invention that the contents of the risk factors obtained through the above-mentioned 'risk estimation' processing part A are not shown merely as a collection of data, but rather an 'overall risk', i.e. the risk possibility of the test subject contracting an oral disease or the chance of a disease already contracted by the test subject progressing ( this overall risk is generally referred to merely as the 'risk', but here it is referred to as the 'overall risk'), is predicted based on the contents of these numerous risk factors.

To carry out the overall risk judgement based on the contents of the various risk factors obtained through the data gathering part A1, firstly it is necessary to judge which of the risk factors contribute to the overall risk for the test subject. This is because it is not necessarily the case that all of the candidate risk factors act as risk factors for the test subject in question.

Whether or not each of the risk factors contributes to the overall risk, and in the case that a risk factor does contribute to the overall risk the extent of this contribution (i.e. the risk value), is determined in an unequivocal fashion by referring to the risk value judgement criteria section A21. In some cases there is one judgement criterion for one risk factor, but in some cases a judgement criterion is set in an overall fashion for two or more risk factors. The 'eating habits' item is an example of the latter.

The basic examination in the data gathering part A1 refers to an examination for which the involvement of a dentist, a dental hygienist or the like is necessary either for the examination itself or for interpreting the examination results, and predominantly involves an examination of the state of the oral cavity or the like. The question-based examination, on the other hand, is an examination in which the test subject is asked to answer questions about aspects of the state of his/her body of which he/she is self-aware. The form usually adopted is that the test subject reads questions written on a sheet of paper, and after understanding the questions, answers the questions by him/herself. However, if it is judged that involvement of a dentist, a dental hygienist or the like is necessary, then such involvement may be permitted.

The contents of the questions must be such that understanding and answering is easy for the patient. For some items the format of the answers section A12 is a straight choice between two alternatives, i.e. 'yes' or 'no', but for some items the choice is from many alternatives.

The risk values for the various risk factors determined by referring to the risk value judgement criteria section A21 are entered into a judgement section A31 provided to the right of the data gathering part A1. The judgement section A31 is provided with places for entering the risk values corresponding to the various examination items in the data gathering part A1, and a place for entering the overall risk value, which is the total of the risk values. Regarding the section for entering the risk values, some of the risk values are provided with a one-to-one relationship with examination items, but in some cases a single risk value is allocated after totaling the results for a plurality of examination items. A simple calculation is required for this totaling, and in the present embodiment a memo section for this totaling work is provided to the side of the judgement section A31.

The overall risk value thus obtained is represented in the form of a numerical value, and hence the meaning thereof is difficult to understand for a test subject with no specialist knowledge. In the present embodiment, the overall risk value is thus assigned to one of three levels, 'high', 'normal' or 'low' depending on the size thereof, thus giving a final risk judgement, such that the extent of the risk can be understood even by someone other than a specialist. Here, three levels were used, but the number of levels is not limited to this.

The risk judgement result can be represented in words with a plurality of levels as in the present example, but various other forms can be adopted; for example, if perceptualization means such as a diagram with a plurality of levels of display is used, and moreover the diagram is color-coded according to the level of the risk, then a more intuitive grasp of the result becomes possible. Other perceptualization means can also be adopted, for example use of a sheet of paper giving an explanation, a video, a panel, a book, computer software or the like can be envisaged.

The 'risk avoidance measure presentation' processing part B, which is provided extending across sheet 3 and sheet 4, is provided with a risk avoidance measure display part B1 and an improvement possibility display part B2. The 'risk avoidance measure presentation' processing part B is a part for presenting avoidance means that the test subject should currently adopt to avoid disease contraction or progression, in accordance with the final risk judgement result. The improvement possibility display part B2, on the other hand, is a part for displaying the extent to which improvement will be obtained if the avoidance measures are implemented.

The risk avoidance measure display part B1 is provided with a risk avoidance measure presentation criteria section B13 in which are shown the necessity of re-examination and the time of re-examination (recall judgement), a guidance display section B12 in which are displayed guidance contents for guiding lifestyle habits so as to reduce the risk, and a means display section B11 in which are displayed means necessary for carrying out the risk improvement in accordance with the guidance. Tools useful for improving the risk are displayed in the means display section B11. Optimum guidance with regard to avoiding or improving the risk at the present time is displayed in the guidance display section B12. In addition to general guidance, the guidance includes guidance contents that consider the individual situation of the test subject.

The existence of the improvement possibility display part B2 is extremely important in terms of giving the test subject a desire for improvement and maintaining this desire. This is because, by a specific image of improvement being shown, it is possible to maintain effort towards improvement with this image as a goal.

The procedure for filling in the risk improvement table is as follows.

For example, if the answer to the question '⑥ Do you currently take medicine for asthma?' is 'yes', then referring to the corresponding place in the 'item no.' column in the risk value judgement criteria section A21, an allocated risk value of 3 is obtained, and this value is entered in the corresponding place in the judgement section A31. Note that the format for entering the risk value into the judgement section A31 may be to directly enter the number '3' in the place in question as in the present example, or a format may be adopted in which a plurality of possible choices are written in advance in the judgement section A31 and a mark is placed against the correct choice.

The other questions are answered in the same way and each of the risk values is filled in. Note, however, that there are cases in which a single risk value is allocated in an overall fashion for a plurality of risk factors, as in the case of 'eating habits' in the risk value judgement criteria section A21. FIG. 2 indicates that '2' is allocated as the risk value if the total of the answers to examination items ⑦, ⑧, ⑨ and 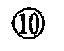 is 5 or more. The reason for this is that 'eating habits' is not of the nature that the risk value thereof is determined by a single examination item, but rather a risk value is only allocated once a plurality of examination items have been compounded with one another.

Once all of the questions have been answered, and all of the risk values corresponding to the questions have been filled into the judgement section A31, the risk values are added together and the total thereof is entered into the bottom row of the judgement section A31. This total is the overall risk value, and represents, in the form of a numerical value, the risk of the test subject contracting the specified disease (for example, 'caries'), or in the case that the disease has already been contracted, the possibility of the disease progressing. This numerical value can be understood by a specialist, but may be hard to understand for the test subject. In the present embodiment, so that the test subject will be able to gain an intuitive grasp of the risk of disease, the overall risk value is thus replaced by the expression 'low' if the overall risk value is in a range of 1 to 5, 'normal' if the overall risk value is in a range of 6 to 10, and 'high' if the overall risk value is in a range of 11 to 15, thus finally representing the risk of disease as one of three levels. The section showing this relationship between the overall risk and the three levels, i.e. the judgement criteria, is the overall risk judgement criteria section A41 provided on sheet 2 (FIG. 2). Moreover, the level of the risk for the test subject is represented in the form of an easy-to-understand word or phrase, picture or the like in the overall risk display section A42 provided on sheet 3 (FIG. 3). Here, three levels were set, but the number of levels may be more than this. Moreover, not only is the overall risk displayed, but also an explanation section A43 for giving a more detailed explanation is provided. It is extremely important for the test subject to have detailed and correct knowledge of the risk from the standpoint of implementing risk avoidance measures as described below. In addition to words and phrases, the explanation section A43 may contain diagrams, graphs, pictures and the like. Moreover, utilization of a video tape, CD-ROM or the like also falls under the category of the explanation section A43.

As a result of filling in the risk improvement table following the procedure described above, the risk of the test subject at the present time towards the specified disease can be expressed in an easy-to-understand way, and hence the test subject can understand what kind of state he/she is in objectively and accurately. Moreover, at the same time as giving an analysis of the current state in this way, the risk improvement table also presents specific measures for preventing or reducing the risk in an easy-to-understand way, and hence by improving one's lifestyle following these measures the risk can be improved.

The risk improvement table may be provided alone, but if the risk improvement table is presented as part of a risk care set in combination with various risk improvement tools, then the risk improvement effects can be further heightened. Principal examples of risk improvement tools are tools used for guidance, and products for the oral cavity such as medicaments for the oral cavity and implements for the oral cavity. Guidance tools are used for the purpose of teaching knowledge, increasing awareness and the like with regard to oral hygiene, diseases and the like, and include, for example, videos, CDs, cassette tapes, panels, books, computer software and the like containing guidance on improving eating habits, brushing guidance and the like. When a guidance tool is combined with a computer, either a general-purpose computer may be used, or else a specialist device in which, for example, the guidance tool is integrated with a display may be used.

Products for the oral cavity include mouthwashes, sprays, foams, gels, tablets, chews, capsules, gum, foods, toothbrushes, interdental brushes, dental floss, and irrigators, with specific examples including foams for strengthening dentine, varnishes for preventing bacterial infection, foods for strengthening teeth and bones, chews for preventing mouth dryness, capsules for reducing damage due to smoking, tablets that supplement components in the saliva, dental floss for killing bacteria in specific areas, interdental brushes for removing plaque from specific areas, irrigators for washing specific areas, and capsules for improving the patient's resistance. When presenting the risk improvement tools to the patient, there is social significance in recommending specific products of specific companies, for example '... made by ...', thus striving to popularize excellent products.

Moreover, the risk improvement table and the risk improvement tools may be combined with examination means for judging risk factors, thus constructing a system for carrying out risk care business. Such business can be said to have demands from society as a backdrop.

An example of the risk improvement table of the present invention has been described above. However, it should be noted that other arrangements of the various sections and display forms can be adopted as appropriate. Moreover, various applications of the risk improvement table of the present invention can be envisaged, so long as this is within a scope such that the purport of the invention is not deviated from. For example, the whole of the risk improvement table may be realized on a computer. In this case, the various display sections of the risk improvement table may be displayed on a display screen, and entry into the display sections may be carried out using a pointing device such as a mouse or a touch panel or voice input means, supplemented by a keyboard.

Moreover, one can also envisage taking the contents of the explanation section and the guidance display section and contents introducing the risk improvement tools from a database stored on a storage medium such as a CD-ROM or an HDD.

Furthermore, a computer on which is installed software for the risk improvement table may be connected to a host computer via a communications network, so that the contents of the explanation section and the guidance display section and contents introducing the risk improvement tools can be provided from the host computer, and a specialist's judgement can be partially reflected in the risk judgement. In this case, updating of the contents can be handled on the host computer side, and hence the latest risk improvement measures can be provided. Moreover, by placing a simple terminal device on which is installed software for the risk improvement table in a household, and executing improvement guidance measures, it is possible to build up a history of the state of health of the oral cavity, which changes with time, on a host computer via a communication line, thus improving the accuracy of risk judgement.

### [Industrial applicability]

The risk improvement table disclosed in claim 1 is provided with a 'risk estimation' processing part and a 'risk avoidance measure presentation' processing part. As a result, the risk improvement table can be used in risk judgement, and at the same time specific measures for avoiding or improving the judged risk can be presented.

If, as well as a judgement criteria part that shows risk judgement criteria for the individual risk factors, the 'risk estimation' processing part is also provided with an overall risk display part as in claim 2, then a judgement result of the overall risk at the present time for the test subject towards the specified disease can be indicated. As a result, it becomes possible to calculate an overall risk value that does not depend on the experience or knowledge of the person making the judgement, and moreover even if this person does not have specialist knowledge.

If the display contents of the overall risk display part are represented in a multi-level format as in claim 3, then the risk at the present time towards the specified disease can be displayed so as to be intuitively understandable even by someone who is not a specialist.

If an improvement possibility display part is provided as in claim 4, then a display that makes it easy to be enthusiastic about carrying out risk avoidance or improvement becomes possible.

If part or the whole of the 'risk estimation' processing part is realized through a computer program as in claim 5, then the effort required to fill in the table and carry out the processing necessary for the risk judgement can be reduced, and the interest of the test subject can be engaged.

If the risk improvement table is applied to the field of oral medicine as in claim 6, then the risk of an oral disease being contracted or progressing can be predicted, and moreover specific measures for suppressing the contraction or progression of the oral disease can be indicated.

If a risk improvement method using the above-mentioned risk improvement table is adopted as in claim 7, then objective judgement of risk and presentation of risk avoidance measures can be carried out simply by carrying out sequential processing in accordance with the display contents of the risk improvement table,

If a risk care set in which are combined the above-mentioned risk improvement table and risk improvement tools is constructed as in claim 8, or a risk improvement method using such a risk care set is adopted as in claim 9, then more specific proposals targeting everything from risk judgement to risk improvement can be given.

By following the procedure disclosed in claim 10, risk improvement tables for various diseases can be created.

If a risk care business system as in claim 11 is constructed, then a comprehensive service from risk judgement to specific guidance for risk avoidance can be carried out.

## Claims

1. A risk improvement table, comprising:
a risk estimation processing part; and
a risk avoidance measure presentation processing part.

2. The risk improvement table according to claim 1, wherein said risk estimation processing part contains a judgement criteria part and an overall risk display part.

3. The risk improvement table according to claim 1 or claim 2, wherein the overall risk display part is represented in a multi-level format.

4. The risk improvement table according to any one of claims 1 through 3, wherein an improvement possibility display part is provided.

5. The risk improvement table according to any one of claims 1 through 4, wherein part or the whole of said risk estimation processing part and said risk avoidance measure presentation processing part are realized through a computer program.

6. The risk improvement table according to any one of claims 1 through 5, wherein the risk improvement table is applied to a field of oral medicine.

7. A risk improvement method using the risk improvement table according to any one of claims 1 through 6.

8. A risk care set, comprising the risk improvement table according to any one of claims 1 through 6, and risk improvement tools.

9. A risk improvement method using the risk care set according to claim 8.

10. A risk improvement table creation method of creating the risk improvement table according to any one of claims 1 through 6, wherein said risk estimation processing part is created through a sequence of operations comprising:
identifying principal risk factors for a targeted oral disease;
eliminating ones of the identified risk factors that are known to be correlated to other risk factors;
calculating risk values, which represent rates of contribution to disease outbreak, for the risk factors remaining after the elimination operation;
establishing a relationship between an overall risk value calculated from the risk values for the plurality of risk factors and a state of progression of the oral disease; and
identifying question-based examination items and oral cavity examination items necessary for judging whether or not a test subject has the risk factors, and as far as possible replacing ones of the identified question-based examination items and oral cavity examination items that are unsuitable for implementation in a clinical setting with other items correlated thereto, thus adjusting the items so that only items suitable for implementation in a clinical setting remain.

11. A risk care business system, comprising:
the risk improvement table according to any one of claims 1 through 6, created based on the risk improvement table creation method according to claim 10;
examination means for implementing a question-based examination and an oral cavity examination as indicated by said risk improvement table on each test subject;
calculating means for applying examination results to said risk estimation processing part of said risk improvement table, working out a risk value for each risk factor judged objectively, and calculating an overall risk value; and
risk improvement tools corresponding to avoidance measures presented by said risk avoidance measure presentation processing part of said risk improvement table.
